# EUROPEAN PATENT APPLICATION

(11) **EP 3 815 604 A1**
(43) Date of publication of application: **05.05.2021**
(21) Application number: 19826479.8
(22) Date of filing: 12.06.2019
(51) Int. Cl.: A61B 5/026, A61B 5/11, A61B 5/22, A63B 69/00

(54) **BLOOD FLOW ANALYSIS DEVICE, BLOOD FLOW ANALYSIS PROGRAM, AND BLOOD FLOW ANALYSIS SYSTEM**

(30) Priority: 29.06.2018 JP 2018123983
(71) Applicant: Hitachi High-Tech Corporation, Minato-ku Tokyo 105-6409 (JP)
(72) Inventor: TANAKA, Takeshi, Tokyo 100-8280 (JP); KURATA, Sayaka, Tokyo 100-8280 (JP); TOYOMURA, Takashi, Tokyo 100-8280 (JP)
(74) Representative: Strehl Schübel-Hopf & Partner
(86) International application number: PCT/JP2019/023177
(87) International publication number: WO 2020/004020

(57) **Abstract**

The present invention aims to provide a blood flow analysis device capable of accurately identifying a change in the blood flow in a muscle caused by resistance exercise performed by a subject. A blood flow analysis device according to the present invention specifies a resistance exercise period during which a muscle of a subject present at a position to be measured by a motion sensor is performing a resistance exercise, and analyzes a light detection signal in the resistance exercise period, to thereby calculate a feature amount of a change in the blood flow in the muscle of the subject in the resistance exercise period (see FIG. 1).

## Description

### Technical Field

The present invention relates to a technique for analyzing the blood flow in a muscle of a subject.

### Background Art

Hemoglobin in the human blood absorbs near infrared light. Therefore, when near infrared light is radiated into the human body, the reflection amount of the near infrared light changes according to a change in the blood flow rate. Utilizing this property, it is possible to measure brain activity and muscle activity in a non-invasive manner, by irradiating the brain with near infrared light from the outside, measuring the reflected near infrared light, and analyzing the amount of received light. Such a measuring device is called a near infrared spectroscopy device (NIRS). For example, for the purpose of fitness, it is expected that the exercise effect can be simply measured by utilizing NIRS in the exercise such as strength training.

PTL 1 mentioned below discloses a technique for measuring the exercise effect by optically measuring the oxygen concentration of muscle blood. The literature aims to "provide an exercise assisting device that contributes to improvement of the training effect." In order to achieve the aim, "the exercise assisting device 1 includes a control device 30 configured of a plurality of electrical elements. The control device 30 acquires a measurement signal from a measurement unit 10 that outputs a measurement signal reflecting the oxygen concentration of the blood that supplies oxygen to the muscle during the training in which a first state where the muscle is loaded and a second state where the muscle is not loaded are repeated. Then, the control device 30 causes a notifying unit 22 to output exercise information including the exercise resumption time that is the time when the second state shifts to the first state, on the basis of the measurement signal." (see Abstract).

### Citation List

### Patent Literature

PTL 1: JP 2016-214309 A

### Technical Problem

In order to understand the metabolic activity and the like in the muscle based on a change in the blood flow in the muscle during exercise, it is necessary to extract a blood flow change pattern at each time point such as before exercise/during exercise/after exercise, and analyze the increase/decrease characteristics. However, conventionally, it has been usual to perform extraction thereof by visual observation, and the human cost for it has been a problem.

The art described in PTL 1 uses the oxygen concentration of blood in the muscle to estimate an exercise start time point, an exercise end time point, and the like. However, since the oxygen concentration of blood in the muscle may change due to factors other than exercise, the accuracy of estimation of the exercise start time point, the exercise end time point, and the like may not necessarily be high, depending on the condition of the subject. Therefore, it is considered that the technique described in the literature still has a problem that is the same as the conventional one.

The present invention has been made in view of the above problem. An object of the present invention is to provide a blood flow analysis device capable of accurately identifying a change in the blood flow in the muscle caused by resistance exercise performed by a subject.

### Solution to Problem

A blood flow analysis device according to the present invention specifies a resistance exercise period during which a muscle of a subject, present at a position to be measured by a motion sensor, is performing a resistance exercise, and analyzes a light detection signal in the resistance exercise period, to thereby calculate a feature amount of a change in the blood flow in the muscle of the subject in the resistance exercise period.

### Advantageous Effects of Invention

According to the blood flow analysis device of the present invention, it is possible to accurately specify a period during which the subject is performing a resistance exercise. Thereby, it is possible to accurately identify a change in the blood flow in the muscle caused by the resistance exercise performed by the subject.

### Brief Description of Drawings

[FIG. 1] FIG. 1 is a configuration diagram of a blood flow analysis system 10 according to a first embodiment.
[FIG. 2] FIG. 2 is a functional block diagram showing a configuration of a sensor 300.
[FIG. 3] FIG. 3 is a diagram illustrating an object to be measured by an acceleration/angular rate sensor 305.
[FIG. 4] FIG. 4 is a functional block diagram of an analysis terminal 100.
[FIG. 5A] FIG. 5A illustrates an example of a motion measurement signal measured by the acceleration/angular rate sensor 305 in a leg press exercise.
[FIG. 5B] FIG. 5B illustrates an example of a motion measurement signal measured by the acceleration/angular rate sensor 305 in a squat exercise.
[FIG. 6] FIG. 6 is a flowchart illustrating a procedure of calculating a feature amount of the muscle blood flow of a subject 400 by an analysis program 111.
[FIG. 7] FIG. 7 is a graph exemplifying a feature amount of the blood flow in the muscle of the subject 400.
[FIG. 8] FIG. 8 is an example of an exercise effect index displayed on the display 140.

### Description of Embodiments

### <First Embodiment>

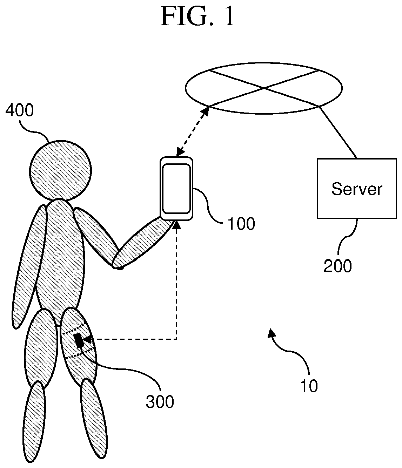

FIG. 1 is a configuration diagram of a blood flow analysis system 10 according to a first embodiment of the present invention. The blood flow analysis system 10 is a system that analyzes a change in the blood flow in the muscle of a subject 400 when the subject 400 is performing a resistance exercise. The blood flow analysis system 10 has an analysis terminal 100, an analysis server 200, and a sensor 300.

The sensor 300 is attached on the muscle of the subject 400. The sensor 300 acquires a motion measurement signal that represents the motion of the muscle of the subject 400, and acquires a light measurement signal that represents a change in the blood flow in the muscle of the subject 400. The analysis terminal 100 acquires each measurement signal from the sensor 300, and uses the measurement signal to calculate a feature amount that represents a change in the blood flow in the muscle when the subject 400 is performing a resistance exercise. The analysis server 200 can collect the calculation results from the analysis terminal 100 and perform another analysis process based on the calculation results of a plurality of subjects 400. For example, it is possible to calculate an average blood flow change tendency of a plurality of subjects 400.

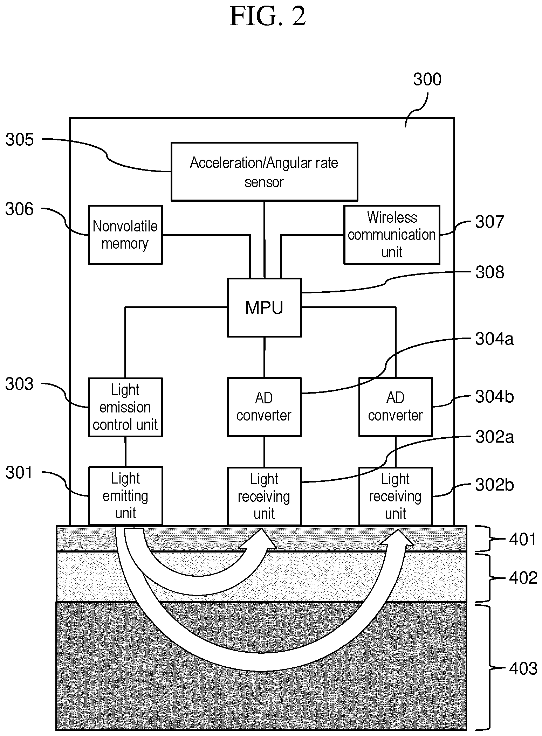

FIG. 2 is a functional block diagram showing the configuration of the sensor 300. The sensor 300 includes a light emitting unit 301, light receiving units 302a and 302b, a light emission control unit 303, AD converters 304a and 304b, an acceleration/angular rate sensor 305, a nonvolatile memory 306, a wireless communication unit 307, and a processor 308.

The light emitting unit 301 is attached so as to contact the skin 401 of the subject 400, and irradiates the muscle of the subject 400 with near infrared light. The light receiving units 302a and 302b receive the near infrared light reflected from the muscle of the subject 400, and output light measurement signals indicating the intensity thereof. The light receiving unit 302a is disposed on the skin 401 at a position closer to the light emitting unit 301 than the light receiving unit 302b. Therefore, the light receiving unit 302a receives a large amount of near infrared light reflected from a relatively shallow position, and the light receiving unit 302b receives a large amount of near infrared light reflected from a relatively deep position. For example, a measurement result by the light receiving unit 302a includes a large amount of light components that have passed through fat 402, and a measurement result by the light receiving unit 302b includes a large amount of light components that have passed through muscle 403.

The acceleration/angular rate sensor 305 measures the acceleration and the angular rate at a site where the sensor 300 is attached to thereby measure the motion of the muscle at the site, and outputs a motion measurement signal representing the measurement result. The acceleration/angular rate sensor 305 can be configured using a gyro sensor, for example. A specific exemplary configuration of the acceleration/angular rate sensor 305 will be described later.

The light emission control unit 303 controls the light emitting unit 301. The AD converters 304a and 304b each convert measurement signals output by the light receiving units 302a and 302b into digital values. The processor 308 collects the motion measurement signals acquired by the acceleration/angular rate sensor 305 and the light measurement signals acquired by the light receiving units 302a and 302b, and transmits them to the analysis terminal 100. The nonvolatile memory 306 stores data to be used by the processor 308. The wireless communication unit 307 is a communication interface that wirelessly transmits each measurement signal collected by the processor 308 to the analysis terminal 100.

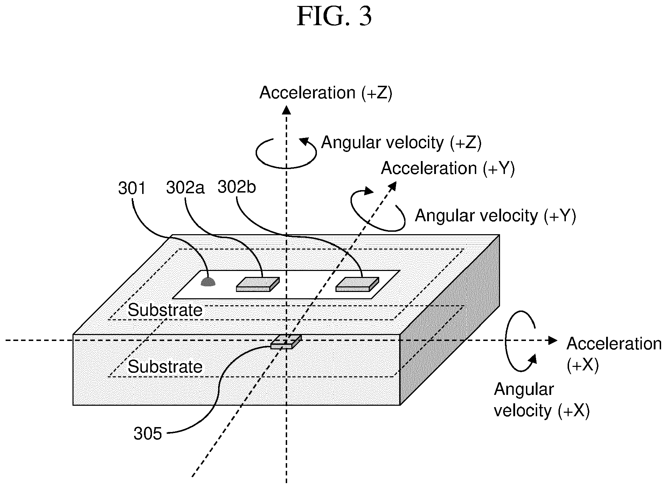

FIG. 3 is a diagram for explaining a measurement target by the acceleration/angular rate sensor 305. The acceleration/angular rate sensor 305 measures acceleration around each of the XYZ axes and also measures rotation around each of the XYZ axes.

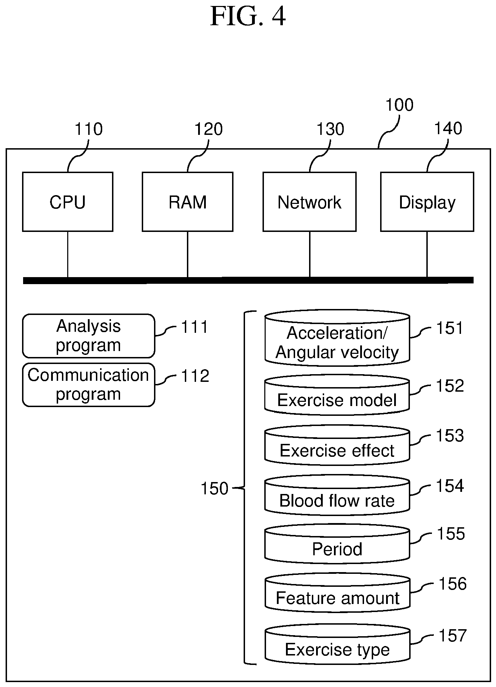

FIG. 4 is a functional block diagram of the analysis terminal 100. The analysis terminal 100 includes a central processing unit (CPU) 110, a random access memory (RAM) 120, a network interface 130, a display 140, and a storage device 150. These are interconnected by communication lines. The analysis terminal 100 can be configured of a device such as a smartphone.

The CPU 110 executes an analysis program 111 and a communication program 112. For convenience of description, these programs may be described below as operating subjects, but the CPU 110 executes these programs actually. The analysis program 111 and the communication program 112 can be stored in the storage device 150, for example.

The analysis program 111 specifies a resistance exercise period of the subject 400 using the motion measurement signal acquired by the acceleration/angular rate sensor 305, and calculates the feature amount of the muscle blood flow in the resistance exercise period using the light measurement signals acquired by the light receiving units 302a and 302b. The specific procedure will be described later. The communication program 112 communicates with an external device (for example, the analysis server 200) via the network interface 130.

The RAM 120 temporarily holds the data to be used by the CPU 110. The display 140 displays the calculation result by the CPU 110 on the screen. For example, it is possible to display a screen interface that presents an exercise effect index described below.

The storage device 150 stores acceleration/angular rate data 151, exercise model data 152, exercise effect data 153, blood flow rate data 154, period data 155, feature amount data 156, and exercise type data 157. Details of these types of data will be described together with the operation of the analysis program 111.

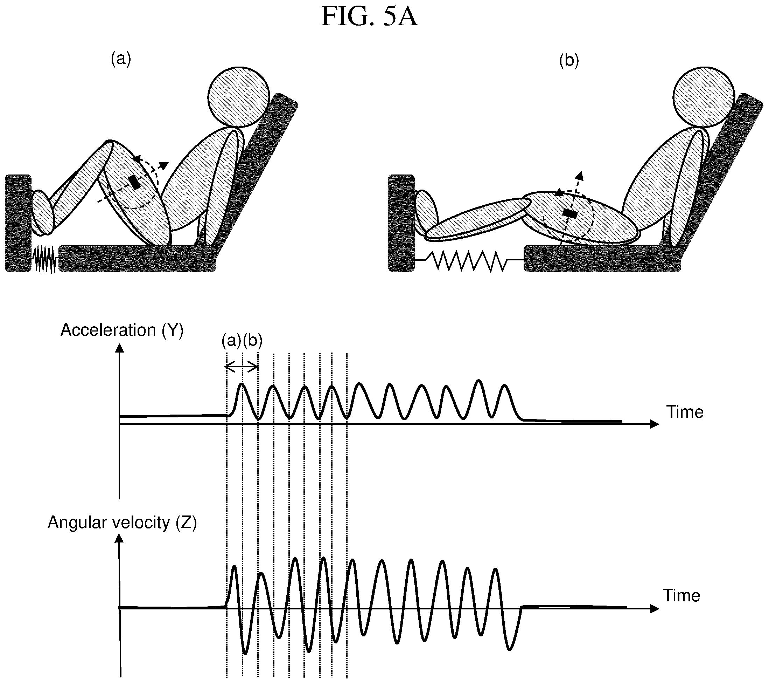

FIG. 5A is an example of a motion measurement signal measured by the acceleration/angular rate sensor 305 in the leg press exercise. The leg press exercise consists of repetition of a step (a) of relaxing the thigh muscle of the subject 400 and a step (b) of contracting the muscle. When the sensor 300 is attached to the outside of the thigh of the subject 400, the acceleration in the Y-axis direction and the angular rate around the Z-axis described in FIG. 3 change with time as illustrated in FIG. 5A.

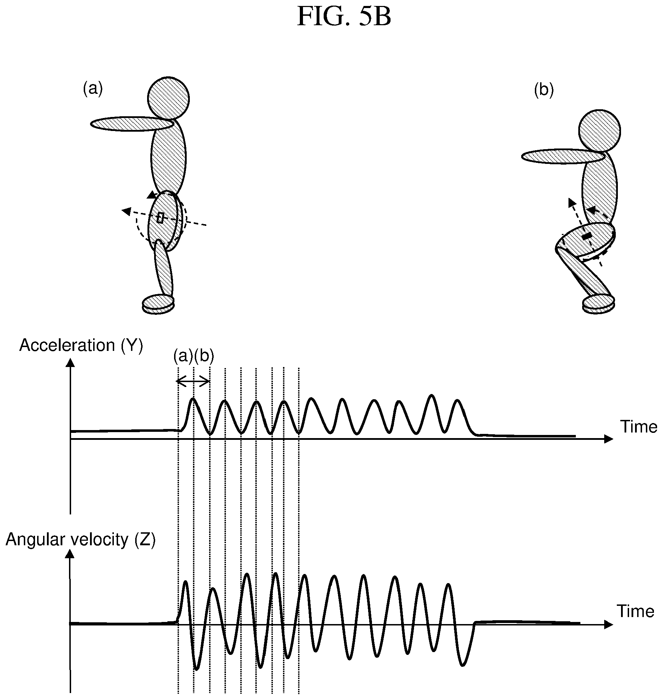

FIG. 5B is an example of a motion measurement signal measured by the acceleration/angular rate sensor 305 in the squat exercise. The squat exercise consists of repetition of a step (a) of relaxing the thigh muscle of the subject 400 and a step (b) of contracting the muscle. When the sensor 300 is attached to the outside of the thigh of the subject 400, the acceleration in the Y-axis direction and the angular rate around the Z-axis described in FIG. 3 change with time as illustrated in FIG. 5B.

The patterns of changes over time of acceleration and angular rate illustrated in FIGS. 5A and 5B can be categorized to some extent for each exercise type. It can be said that such tendency is strong particularly in an exercise type in which a load is applied to a muscle of a specific site of the subject 400 such as a resistance exercise. The exercise model data 152 describes the patterns of changes over time of acceleration and angular rate for each exercise type of such resistance exercise. The analysis program 111 can estimate the type of resistance exercise performed by the subject 400 by comparing the motion measurement signal acquired from the sensor 300 with the exercise model data 152.

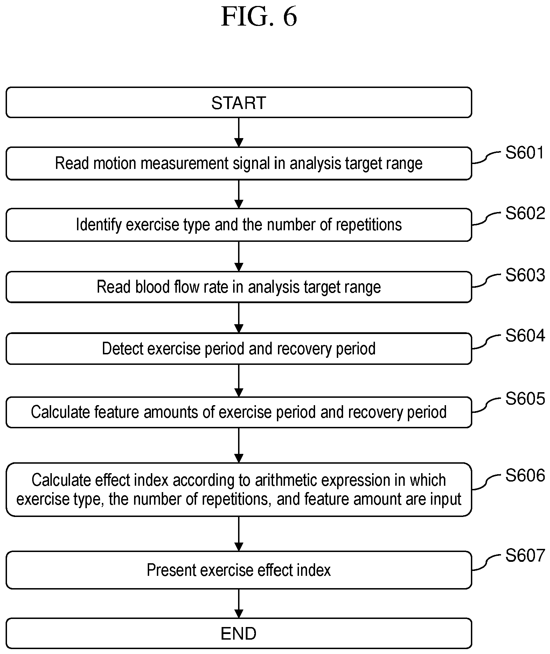

FIG. 6 is a flowchart illustrating a procedure of calculating the feature amount of the muscle blood flow of the subject 400 by the analysis program 111. Each step of FIG. 6 will be described below.

### (FIG. 6: Step S601)

During the time when the subject 400 is performing a resistance exercise, the acceleration/angular rate sensor 305 acquires motion measurement signals. The communication program 112 acquires the motion measurement signals from the sensor 300 and stores them as the acceleration/angular rate data 151. The analysis program 111 reads the motion measurement signals within the analysis target range from the acceleration/angular rate data 151. The analysis target range is, for example, the date when the muscle blood flow of the subject 400 is to be analyzed.

### (FIG. 6: Step S602)

The analysis program 111 compares the acceleration/angular rate data 151 read in step S601 with the exercise model data 152 to thereby identify the exercise type and the number of repetitions of the subject 400 described in the acceleration/angular rate data 151. The number of repetitions can be identified by, for example, recognizing the exercise type and extracting a motion pattern of one repetition unit, and counting the number of times thereof. The analysis program 111 stores the identified exercise type and the number of repetitions as the exercise type data 157.

### (FIG. 6: Step S603)

The analysis program 111 reads, from the blood flow rate data 154, a change over time in the blood flow rate within the same analysis target range as in step S601. In the processing of the subsequent steps, it is necessary to synchronize the motion measurement signal and the light measurement signal. That is, it is necessary to associate both types of data of the same time. For example, when the time scales of the two types of data are different, the analysis program 111 may appropriately perform processing for aligning them.

### (FIG. 6: Step S604)

The analysis program 111 detects, from the motion measurement signals read in step S601, an exercise period during which the subject 400 is performing the resistance exercise and a recovery period in which the subject 400 suspended the exercise and takes a rest. The exercise period can be detected at both the start time point and the end time point according to whether or not the change rate in each of the acceleration and the angular rate exceeds a threshold value. The start time point of the recovery period may be the same as the end time point of the exercise period. The end time point of the recovery period will be described in the step described later. The analysis program 111 stores data (for example, the time) that specifies each detected period as period data 155.

### (FIG. 6: Step S605)

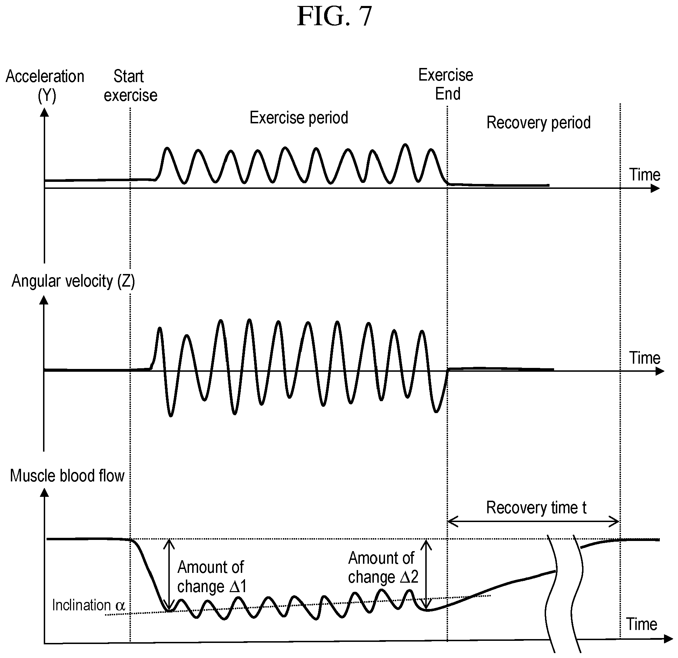

The analysis program 111 calculates the feature amount of a change in the blood flow in the exercise period and the feature amount of a change in the blood flow in the recovery period, respectively. Examples of these feature amounts will be described again with reference to FIG. 7 described later. The analysis program 111 stores the calculated feature amount as the feature amount data 156.

### (FIG. 6: Step S606)

The analysis program 111 calculates an effect index indicating the effect of the resistance exercise performed by the subject 400 by using the exercise type/number of repetitions/feature amount. For example, it is conceivable to convert these measured values into an effect index by substituting these measured values into an arithmetic expression configured as a function of the exercise type/number of repetitions/feature amount. Furthermore, for example, for other subjects of the same age as the subject 400, it is also possible to calculate effect indexes using the same function and store the average value thereof in the storage device 150 in advance, and compare the effect index calculated in this step with the average value to thereby calculate a relative effect index. For example, when the time until the blood flow settles is longer than the average of the same age group, it can be said that the subject 400 exercised with a higher intensity, and thus the exercise effect is high.

### (FIG. 6: Step S607)

The analysis program 111 presents the calculated effect index to the subject 400 on the display 140. An example of a screen display in this step will be described again with reference to FIG. 9 described later.

FIG. 7 is a graph exemplifying the feature amount of the blood flow in the muscle of the subject 400. The analysis program 111 can calculate the blood flow rate in the muscle of the subject 400 according to a light detection signal measured by the sensor 300 at an appropriate time before starting the flowchart of FIG. 6, and store it as the blood flow rate data 154. In step S605, the analysis program 111 can obtain Δ1, Δ2, α, and t illustrated in FIG. 7 as feature amounts. Each feature amount will be described below.

Δ1 represents a difference between the muscle blood flow rate before the subject 400 starts the resistance exercise and the muscle blood flow rate during the resistance exercise. Since the muscle blood flow rate during the exercise period changes with time, for example, a difference between the muscle blood flow rate peak in the exercise period and the muscle blood flow rate before the start can be obtained as Δ1. Alternatively, a difference between α, described below, and the muscle blood flow rate before the start can be obtained as Δ1 instead. The time point for obtaining Δ1 may be any time point during the exercise period.

Δ2 represents a difference between the muscle blood flow rate during the time when the subject 400 is performing the resistance exercise and the muscle blood flow rate at a time point when the blood flow becomes stable in the recovery period. The muscle blood flow rate during the exercise period is the same as that of Δ1. The time point when the blood flow becomes stable in the recovery period may be, for example, a time point when the blood flow rate returns to the muscle blood flow rate before the start of exercise, or a time point when the change rate of the muscle blood flow rate becomes less than a threshold in the recovery period.

α represents the inclination of a straight line that is obtained by smoothing the muscle blood flow rate during the exercise period. When the subject 400 becomes accustomed to the exercise during the exercise period, the muscle blood flow rate may change in a slightly stable direction (or vice versa). It can be said that α indirectly represents such a characteristic of the subject 400. For example, the moving average of the muscle blood flow rate during the exercise period can be obtained as α. Alternatively, the muscle blood flow rate may be smoothed by another suitable method.

t represents a length of time from the start time point of the recovery period to a time point when the blood flow becomes stable. A time point when the blood flow becomes stable in the recovery period can be obtained as described above. Since t indirectly represents how much load the subject 400 receives from the resistance exercise, it can be said that it is suitable as a feature amount.

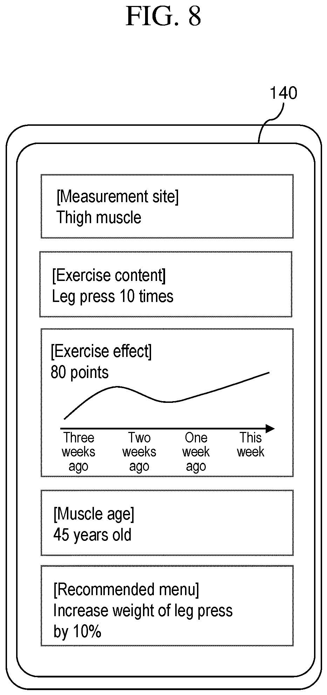

FIG. 8 is an example of an exercise effect index displayed on the display 140. The measurement site can be input to the analysis terminal 100 by the subject 400 himself/herself, for example. The exercise content is the exercise type recognized by the analysis program 111. The exercise effect and the muscle age correspond to an effect index calculated by the analysis program 111, or a relative effect index further calculated based on the effect index. The analysis program 111 can also present a training advice to the subject 400 according to the effect index. For example, the effect index of the subject 400 can be compared with the average of the effect indexes of the same age group, and a message suggesting an increase or decrease of the exercise load can be presented according to the difference. FIG. 8 illustrates an advice when the exercise load is light.

### <First Embodiment: Summary>

The blood flow analysis system 10 according to the first embodiment specifies an exercise period and a recovery period of the subject 400 by the acceleration/angular rate sensor 305, and calculates the feature amounts of a change in the blood flow in the muscle during the specified period. As a result, it is possible to accurately specify the period during which the subject 400 is performing a resistance exercise without a manual work, and then to accurately calculate the effect of the resistance exercise.

The blood flow analysis system 10 according to the first embodiment compares a motion measurement signal acquired by the acceleration/angular rate sensor 305 with the motion model data 152 to thereby estimate the exercise type and the number of repetitions of the resistance exercise performed by the subject 400. As a result, it is possible to save the load of inputting the type of resistance exercise into the analysis terminal 100 by the subject 400. In particular, in the resistance exercise, since it is relatively easy to specify the exercising site and the motion pattern, the estimation accuracy by the analysis program 111 can be increased.

### <Second Embodiment>

In a second embodiment of the present invention, another processing example that can be performed by the analysis program 111 in addition to the first embodiment will be described. Since the configuration of the blood flow analysis system 10 is the same as that of the first embodiment, an additional processing example will be mainly described below.

After starting the recovery period, the subject 400 may start the next exercise before the muscle blood flow rate becomes stable. In that case, the recovery time t described in FIG. 7 cannot be specified. In that case, the analysis program 111 may estimate t according to a change in the muscle blood flow rate from the time when the recovery period is started until the time when the subject 400 resumes the exercise. For example, it is possible to estimate t by calculating the gradient (or average change rate) of the muscle blood flow rate from the time point of starting the recovery period to the time point of resuming the exercise, and linearly interpolating the change over time in the muscle blood flow rate according to the gradient. For example, the time when the muscle blood flow rate is expected to return to the level before the start of the exercise may be regarded as the end time point of the recovery period. Alternatively, t may be estimated by an appropriate interpolation calculation. Whether or not the subject 400 resumed the exercise in the middle of the recovery period can be determined by the same method as that for the time point of starting the exercise period.

In the first embodiment, it has been described that comparison is made between the average of the effect indexes of the same age group as the subject 400 and the effect index of the subject 400 himself/herself. Alternatively, an effect index for each exercise may be calculated based on a change over time in the effect index of the subject 400 himself/herself. For example, every time the analysis program 111 calculates an effect index, the result is accumulated as the exercise effect data 153 and the average value thereof is calculated, and every time the subject 400 performs the exercise, the analysis program 111 compares the effect index of the exercise with the average value of the past effect indexes of the subject 400 himself/herself. Thereby, a relative effect index can be calculated.

An effect index serving as a reference, such as the average effect index of the same age group of the subject 400, can be set by the subject 400 himself/herself. For example, this corresponds to the case where the subject 400 himself/herself determines a target value of the exercise load. In that case, for example, on a screen interface provided by the display 140, the subject 400 inputs at least one of the exercise type/number of repetitions/feature amount and sets an effect index to be used as a comparison reference by himself/herself. For example, a reference value can be set in such a manner that if the leg press exercise/30 times/recovery period of 1 minute, the effect index is 100 points.

Although the first embodiment has described that the analysis program 111 estimates the exercise type and the number of repetitions, in addition, the subject 400 may designate the exercise type to the analysis terminal 100. For example, one or both of the exercise content and the number of times of the exercise may be input on the screen interface described in FIG. 8. The analysis program 111 may correct the estimation result according to the input, or may input only the exercise type before the exercise and estimate only the number of repetitions.

### <Regarding Modifications of the Present Invention>

Note that the present invention is not limited to the above-described embodiments, but includes various modifications. For example, the above-described embodiments are described in detail to explain the present invention in an easy-to-understand manner, and are not necessarily limited to those having all the described configurations. Further, part of the configuration of one embodiment can be replaced with the configuration of another embodiment, and the configuration of another embodiment can be added to the configuration of one embodiment. Moreover, it is possible to add, delete, and replace other configurations for part of the configurations of the respective embodiments.

The process of calculating the feature amount of the blood flow change of the subject 400 may be performed by the analysis server 200. In that case, the analysis terminal 100 has a role of collecting measurement signals from the sensor 300. Further, the arithmetic processing can be shared by the analysis terminal 100 and the analysis server 200.

In the above embodiments, the example in which the sensor 300 includes the acceleration/angular rate sensor 305 has been described. However, depending on the type of resistance exercise performed by the subject 400, only one of an acceleration sensor and an angular rate sensor may be used. For example, in the case where the exercise type can be specified using only the site to which the sensor 300 is attached and the angular rate of the muscle, only an angular rate sensor may be used.

In the above embodiments, four examples in FIG. 7 are illustrated as examples of the feature amounts, but other parameters can also be used as feature amounts. For example, the length of time of an exercise period can be cited as one of the candidates.

### Reference Signs List

10 blood flow analysis system
100 analysis terminal
110 CPU
111 analysis program
151 acceleration/angular rate data
152 exercise model data
153 exercise effect data
154 blood flow rate data
155 period data
156 feature amount data
157 exercise type data
200 analysis server
300 sensor
301 light emitting unit
302a∼302b light receiving unit
305 acceleration/angular rate sensor
400 subject

## Claims

1. A blood flow analysis device that analyzes a blood flow in a muscle of a subject, the device comprising:
a light irradiation unit that irradiates the muscle of the subject with light;
a photodetector that detects the light reflected from the subject;
a motion sensor that measures motion of the muscle of the subject; and
an analysis unit that analyzes a light detection signal acquired through detection of the light by the photodetector and analyzes a motion measurement signal acquired through measurement of the motion of the subject by the motion sensor, wherein
the analysis unit specifies a resistance exercise period during which the muscle of the subject present at a position to be measured by the motion sensor is performing a resistance exercise, according to the motion measurement signal, and
the analysis unit analyzes the light detection signal in the resistance exercise period to thereby calculate a feature amount of a change in the blood flow in the muscle of the subject in the resistance exercise period.

2. The blood flow analysis device according to claim 1, wherein
the motion sensor is a sensor that measures at least one of acceleration and angular rate of the muscle of the subject,
the blood flow analysis device further comprises a storage device that stores exercise model data describing a correspondence relationship between a change pattern of the acceleration or the angular rate of the muscle and an exercise type, and
the analysis unit compares at least one of the acceleration and the angular rate of the muscle of the subject measured by the motion sensor with the exercise model data to thereby estimate the exercise type and a number of repetitions of the resistance exercise.

3. The blood flow analysis device according to claim 2, wherein
the analysis unit specifies the resistance exercise period and specifies a recovery period in which the subject ended the resistance exercise and takes a rest, according to at least one of the acceleration and the angular rate of the muscle of the subject measured by the motion sensor, and
the analysis unit calculates the feature amount in the resistance exercise period and also calculates the feature amount in the recovery period.

4. The blood flow analysis device according to claim 1, wherein
the analysis unit calculates the blood flow rate in the muscle of the subject according to the light detection signal, and the analysis unit calculates, as the feature amount, a difference between the blood flow rate in the muscle of the subject before the subject starts the resistance exercise and the blood flow rate in the muscle of the subject in a period during which the subject is performing the resistance exercise.

5. The blood flow analysis device according to claim 1, wherein
the motion sensor is a sensor that measures at least one of acceleration and angular rate of the muscle of the subject,
the analysis unit specifies the resistance exercise period and specifies a recovery period in which the subject ended the resistance exercise and takes a rest, according to at least one of the acceleration and the angular rate of the muscle of the subject measured by the motion sensor,
the analysis unit specifies a time point when the recovery period ends by specifying a time point when a change rate of the blood flow rate in the muscle of the subject in the recovery period reaches a value less than a predetermined threshold, or a time point when the blood flow rate in the muscle of the subject in the recovery period returns to the blood flow rate before start of the resistance exercise period, and
the analysis unit calculates, as the feature amount, a difference between the blood flow rate in the muscle of the subject in a period during which the subject is performing the resistance exercise and the blood flow rate in the muscle of the subject at a time point when the recovery period ends.

6. The blood flow analysis device according to claim 1, wherein
the analysis unit smooths the blood flow rate in the muscle of the subject in the resistance exercise period to thereby calculate, as the feature amount, a gradient of the blood flow rate in the muscle of the subject in the resistance exercise period with respect to a lapse of time.

7. The blood flow analysis device according to claim 1, wherein
the motion sensor is a sensor that measures at least one of acceleration and angular rate of the muscle of the subject,
the analysis unit specifies the resistance exercise period and specifies a recovery period in which the subject ended the resistance exercise and takes a rest, according to at least one of the acceleration and the angular rate of the muscle of the subject measured by the motion sensor,
the analysis unit specifies a time point when the recovery period ends by specifying a time point when a change rate of the blood flow rate in the muscle of the subject in the recovery period reaches a value less than a predetermined threshold, or a time point when the blood flow rate in the muscle of the subject in the recovery period returns to the blood flow rate before start of the resistance exercise period, and
the analysis unit calculates a length of the recovery period as the feature amount.

8. The blood flow analysis device according to claim 1, wherein
the motion sensor is a sensor that measures at least one of acceleration and angular rate of the muscle of the subject,
the analysis unit specifies a recovery period in which the subject ended the resistance exercise and takes a rest, and determines whether or not the subject terminated the recovery period and resumed exercise, according to at least one of the acceleration and the angular rate of the muscle of the subject measured by the motion sensor,
when determining that the subject terminated the recovery period and resumed the exercise, the analysis unit interpolates, at a time after termination, a change in the blood flow rate in the muscle of the subject during a period from a time when the subject started the recovery period until the subject terminated the recovery period to thereby estimate a time point when the blood flow rate in the muscle of the subject in the recovery period returns to the blood flow rate before start of the resistance exercise period, and
the analysis unit calculates a length of time of the recovery period as the feature amount, according to the time point estimated.

9. The blood flow analysis device according to claim 1, wherein
the analysis unit estimates an exercise type of the resistance exercise and a number of repetitions of the resistance exercise according to the motion measurement signal, and
the analysis unit calculates an effect index indicating an effect of the resistance exercise according to the exercise type of the resistance exercise, the number of repetitions of the resistance exercise, and the feature amount.

10. The blood flow analysis device according to claim 9, wherein
the motion sensor is a sensor that measures at least one of acceleration and angular rate of the muscle of the subject,
the analysis unit specifies the resistance exercise period according to at least one of the acceleration and the angular rate of the muscle of the subject measured by the motion sensor, and specifies a recovery period in which the subject ended the resistance exercise and takes a rest, and
the analysis unit calculates the effect index by using the exercise type of the resistance exercise, the number of repetitions of the resistance exercise, the feature amount in the resistance exercise period, and the feature amount in the recovery period.

11. The blood flow analysis device according to claim 10, further comprising
a storage device that stores reference function data describing a reference function serving as a reference for calculating the effect index, as a function of the exercise type of the resistance exercise, the number of repetitions of the resistance exercise, and the feature amount, wherein
the analysis unit calculates the effect index according to a difference between a function value and the reference function, the function value being obtained from the exercise type of the resistance exercise calculated according to the motion measurement signal, the number of repetitions of the resistance exercise calculated according to the motion measurement signal, and the feature amount.

12. The blood flow analysis device according to claim 11, further comprising
an interface that receives an instruction input for modifying the reference function, wherein
the analysis unit modifies the reference function according to the instruction input, and stores the reference function after modification in the storage device as the reference function data.

13. The blood flow analysis device according to claim 1, wherein
the blood flow analysis device includes a first detector and a second detector each as the photodetector,
the first detector is disposed at a position away from the light irradiation unit by a first distance,
the second detector is disposed at a position away from the light irradiation unit by a second distance that is longer than the first distance, and
the analysis unit calculates the blood flow rate in the muscle of the subject according to a difference between the light detection signal detected by the first detector and the light detection signal detected by the second detector.

14. A blood flow analysis program for causing a computer to execute a process of analyzing blood flow in a muscle of a subject, the process including:
a step of acquiring a light detection signal, the light detection signal being acquired by irradiating the muscle of the subject with light and detecting the light reflected from the subj ect;
a step of acquiring a motion measurement signal, the motion measurement signal being acquired by a motion sensor that measures motion of the muscle of the subject; and
an analyzing step of analyzing the light detection signal and the motion measurement signal, wherein
in the analyzing step, the program causes the computer to execute a step of specifying a resistance exercise period in which the muscle of the subject present at a position to be measured by the motion sensor is performing a resistance exercise, according to the motion measurement signal, and
in the analyzing step, the program causes the computer to execute a step of analyzing the light detection signal in the resistance exercise period, thereby calculating a feature amount of a change in the blood flow in the muscle of the subject in the resistance exercise period.

15. A blood flow analysis system comprising:
a computer that executes the blood flow analysis program according to claim 14, and
a server that collects and accumulates analysis results by the computer.
